# EUROPEAN PATENT SPECIFICATION

(11) **EP 1 493 391 B1**
(45) Date of publication and mention of the grant of the patent: **02.12.2009**
(21) Application number: 03745947.6
(22) Date of filing: 07.04.2003
(51) Int. Cl.: A61B 17/12

(54) **ORGAN CONNECTING DEVICE**
ORGANVERBINDUNGSVORRICHTUNG
DISPOSITIF DE CONNEXION D'ORGANE

(30) Priority: 10.04.2002 JP 2002108355
(43) Date of publication of application: 05.01.2005
(73) Proprietor: Yugengaisha Pacs Optica Japan, Misato-shi, Saitama 341-0003 (JP)
(72) Inventor: YAMANOUCHI, Eigoro, Meguro-Ku, Tokyo 152-0022 (JP)
(74) Representative: Kramer - Barske - Schmidtchen
(86) International application number: PCT/JP2003/004407
(87) International publication number: WO 2003/084411

(56) References cited:
- EP-A2- 1 077 047
- JP-B2- 4 074 021
- US-A- 5 690 656

## Description

### Technical Field

The present invention relates to an organ anastomosing apparatus, which is usable to physically expand a narrow through hole (fistula) of an anastomosis portion or constricted portion by causing apoptosis to locally occur around the through hole (fistula) at the narrow region by strongly pinching and pressing with a pair of magnets attracting each other from both sides of the narrow region such as the anastomosis portion or the constricted portion of a gastric or jejunum anastomosis of a subject such as a patient.

### Background Art

In general, the anastomosis of organs such as a gut of a subject such as a patient (which may be described as subject's body hereinafter) is frequently performed to form a bypass (a through hole) between two gut cavities, for example, in order to restore flow of contents of the gut or bile of a bile duct again when constriction of the gut or bile duct progresses due to a tumor, ulcer, inflammation, trauma or the like.

An example of a conventional organ anastomosing apparatus used for such types of anastomosis is described in Japanese Unexamined Patent Application Publication No. HEI 9-10218. In this example, a pair of magnets capable of being automatically self-centered is disposed on both sides of the two organ walls to be anastomosed. By attraction of a pair of large and small magnets, the organ walls are strongly pinched from both sides and are compressed (pressed so as to be pinched) to cause apoptosis to locally occur, thereby forming a through hole (fistula) and the anastomosis, and the peripheral rim (edge) of a small magnet is formed as a sharp cut rim for promoting the anastomosis.

However, in such a conventional organ anastomosing apparatus, the peripheral rim of a small magnet is formed at a sharp cut rim. Thus, there is a concern that other organs may be damaged by the cut rim when this small magnet is inserted into a predetermined organ, inducted into a predetermined area (region), and disposed at the area.

Furthermore, an instrument or apparatus which removes peripheral rims around a narrow through hole (fistula) at an anastomosis portion or constricted portion, so as to physically enlarge the hole, other than by surgical operation means, has not previously been proposed.

US 5,690,656 discloses a device for forming an anastomoses between adjacent viscera, comprising a first magnet having opposing first and second faces, a first jacket having opposing first and second rims, said first jacket formed around a first periphery of the first magnet on a first surface connecting the first and second faces, wherein the first and second jacket rims are spaced farther apart than the first and second magnet faces, wherein said first and second rims include means for promoting tissue necrosis, a second magnet having opposing third and fourth faces, and a second jacket having opposing third and fourth rims, said second jacket formed around a second periphery of the second magnet on a second surface connecting the third and fourth faces, wherein the third and fourth jacket rims are spaced farther apart than the third and fourth magnet faces, wherein the second periphery is smaller than the first periphery, such that the first and second magnets are self-centering when they are magnetically coupled through walls of the adjacent viscera.

EP 1 077 047 A2 discloses an organ anastomosing apparatus, comprising a pair of magnets being disposed to predetermined sites of organs of a subject to be anastomosed to each other so as to be opposed through wall portions of the respective organs, the magnets being adsorbed to each other so as to form an anastomosis site having a through hole for making communication between the organ walls, a flexible guide wire detachably mounted to at least one of the paired magnets, and a guide tube inserted into a body of the subject with said guide wire being inserted therein, said guide tube coming into contact with a guide wire mount surface of said one of magnets so as to support the one magnet when the guide wire is removed from the one magnet, said guide tube being inserted into the through hole of the anastomosis site to maintain formation of the through hole.

The present invention was conceived in view of the circumstances in the related art mentioned above, an object therefore being to provide an organ anastomosing apparatus which is capable of removing peripheral rims around the narrow through hole (fistula) at the anastomosis portion or constricted portion by means of other than surgical operation means, to physically expand the narrow through hole so as to let the anastomosis portion or constricted portion shrink in size.

### Disclosure of The Invention

The present invention is an organ anastomosing apparatus comprising:
a flexible guide wire to be inserted into an organ;
a first magnet formed in a disc shape;
a vinculum (string) secured at a center position of one end surface of the first magnet in an axial direction thereof; and
a second magnet provided with a through hole in which the vinculum is inserted; characterized in that the apparatus further comprises

a moving member for moving the first and second magnets, and that the first magnet is provided with a radial through hole so as to slidably insert the guide wire.

In such organ anastomosing apparatus, it may be desired that the first magnet is provided with a latch member for engaging a turn-around portion of the vinculum when the vinculum is folded in two portions.

In such organ anastomosing apparatus, it may be desired that the vinculum is secured to a center portion of one end surface of the first magnet in an axial direction thereof.

In such organ anastomosing apparatus, it may be desired that the vinculum is made of a material which is dissolved by humor in the organ of a subject.

In such organ anastomosing apparatus, it may be desired that the first magnet is chamfered at corner portions of end surfaces in the axial direction thereof.

In such organ anastomosing apparatus, it may be desired that the moving member is composed of a tubular member movably mounted to the guide wire, said tubular member pushing front end portions of lateral circumferential sides of the first and second magnets.

In such organ anastomosing apparatus, it may be desired that either one of the first and second magnets is provided with a marker made of an X-ray non-transmitting material indicating a magnetic pole of the magnet.

### Brief Description of The Drawings

Fig. 1 is a perspective view showing an essential portion of an organ anastomosing apparatus according to an embodiment of the present invention.
Fig. 2 is a longitudinal sectional view showing a state in a case where the first magnet of the organ anastomosing apparatus shown in Fig. 1 is moved to one side of a constricted portion in an organ.
Fig. 3 is a longitudinal sectional view of the essential portion when inserting the first magnet shown in Fig. 1 into the fistula of the constricted portion.
Fig. 4 is a longitudinal sectional view of the essential portion when moving the first magnet shown in Fig. 1 to the front side of the fistula of the constricted portion.
Fig. 5 is a longitudinal sectional view of the essential portion showing a state after removing the tube shown in Fig. 4 from a guide wire.
Fig. 6 is a longitudinal sectional view of the essential portion showing a state after removing the guide wire shown in Fig. 5 from the first magnet.
Fig. 7 is a longitudinal sectional view of the essential portion showing a state of a second magnet, which has the vinculum of the first magnet inserted through a longitudinal hole, and is moved to the vicinity of the constricted portion, after erecting the first magnet in the organ as shown in Fig. 6.
Fig. 8 is a longitudinal sectional view showing a state when attracting the second magnet shown in Fig. 7 to the first magnet.
Fig. 9 is a perspective view of the essential portion showing a state when pinching and pressing the constricted portion from both sides by the first and second magnets shown in Fig. 8.

### [Reference Numerals in The Drawings]

1 --- organ anastomosing apparatus; 2 --- first magnet; 2atapered portion; 2b --- lateral hole; 2c --- vertical hole; 2dcrossbar; 2e, 2f --- small aperture hole; 2g --- lower hole; 3tube; 4 --- guide wire; 5 --- vinculum.

### Best Mode for Carrying Out The Invention

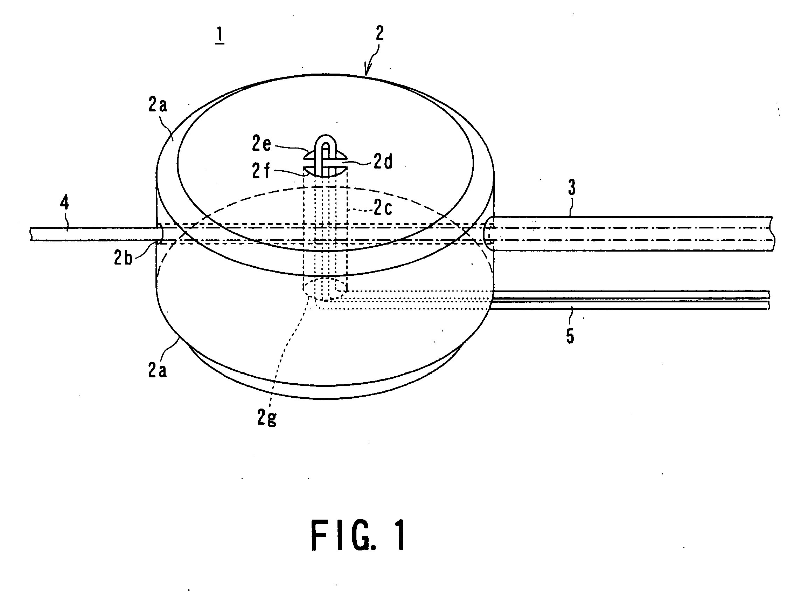

Hereunder, an embodiment of the present invention will be described with reference to Fig. 1 to Fig. 9, in which the same or corresponding elements are designated by the same reference numbers.

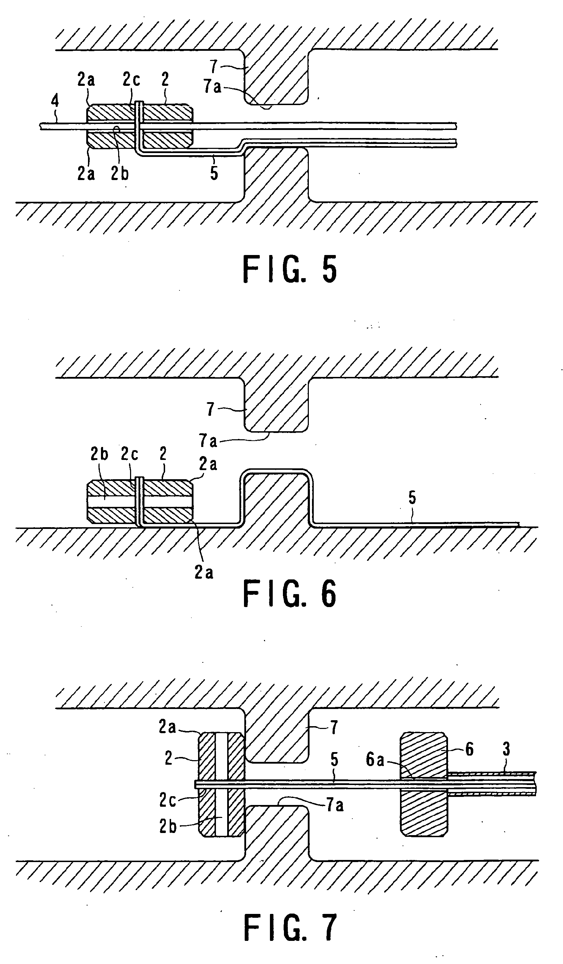

Fig. 1 is a perspective view showing an essential portion of an organ anastomosing apparatus according to one embodiment of the present invention. As shown in Fig. 1, the organ anastomosing apparatus 1 comprises a first magnet 2 made of a rare earth element and formed in a disc shape, transportation means in the form of a tube 3 such as an ileus tube, a guide wire 4 made of a long flexible metal wire to be inserted into an organ of a subject such as a patient, a vinculum 5, and a second magnet 6 formed in a disc-shape as shown in Fig. 7, for example.

The first magnet 2 has a taper (tapered surface) 2a formed on the entire circumferential portion by chamfering corner portions, at both ends, thereof in the axial direction. In addition, the first magnet 2 has a longitudinal hole 2b extending horizontally in the radial direction near the central portion in the axial direction (thickness direction) thereof, and the guide wire 4 is slidably inserted therein.

Furthermore, the first magnet 2 has a vertical hole 2c, as viewed in Fig. 1 (but may be a longitudinal hole 2c as viewed in Fig. 7), extending vertically at the central portions of both end surfaces in the axial direction, and a crossbar 2d is formed so as to connect radial end portions of the vertical hole 2c (top end portion shown in Fig. 1), thus forming a circular-arc-shaped small apertures 2e and 2f at both sides in the width direction of the crossbar 2d.

The thus formed first magnet 2 is coated with at least one of an acid-resistant membrane or a thrombus-preventing membrane on the outer surface thereof, and is provided, at an appropriate portion, with a marker, not shown, made of an X-ray non-transmitting material indicating a magnetic pole.

The tube 3 has an inner diameter larger than that of the guide wire 4 and is formed of a flexible polyvinyl chloride resin or polyurethane resin, for example, so as to provide necessary rigidity for the appropriate amount of push-in response (pushability), torque transmissibility and trackability thereof. Furthermore, it may include an antifriction substance such as silicon oil to provide optimum sliding movement of the guide wire 4.

The push-in response is a characteristic feature which reliably transfers the push-in force from the rear anchor side to the foreend side of the tube 3 when an operator applies a push-in force from the rear anchor side (a gripper side, for example,) to the foreend side in order to move forward the tube 3 in an organ such as the intestine or blood vessels.

Moreover, the above-mentioned torque transmissibility is a characteristic feature which reliably transfers the force rotating around the axis applied from the rear anchor side to the foreend side of the tube 3. Furthermore, the trackability is a characteristic feature which smoothly and reliably makes the tube 3 advance while moving along the guide wire 4 preliminarily inserted in an organ such as a contorted intestine or blood vessels.

The vinculum 5 is inserted, at one end thereof, into the vertical hole 2c of the first magnet 2 from the lower opening 2g so as to extend upward, as viewed in Fig. 1, through the vertical hole 2c. Then, the inserted end extends outward from one small aperture, such as 2e, for example, of the upper opening of the hole 2c. Thereafter, the end extending over the upper opening of the hole 2c is again inserted from the other aperture, such as 2f of the upper opening, into the vertical hole 2c, causing the turn-round point of the vinculum 5 to become latched at the crossbar 2d. The vinculum 5 runs through the vertical hole 2c again and out from the lower opening 2g of the hole 2c so as to extend laterally along the approach route of the vinculum 5 and runs out of the subject's body. At the point where the vinculum 5 intersects at a right angle with the guide wire 4, the approach route and the return route of the vinculum 5 are positioned at different sides in the radial direction of the guide wire 4.

The second magnet 6 may be formed in substantially the same manner as the first magnet 2 so as to have the same size in a disc shape and made of a rare earth element magnet, for example. As shown in Fig. 7, the second magnet 6 has a longitudinal hole 6a extending in the axial direction at the central portion of one axial end surface thereof so as to move along the vinculum 5, which is inserted into the longitudinal hole 6a. Furthermore, the second magnet 6 is coated with at least one of an acid-resistant membrane or a thrombus-preventing membrane on the outer surface thereof. In this regard, however, the second magnet 6 may be either greater or smaller in size than the first magnet 2.

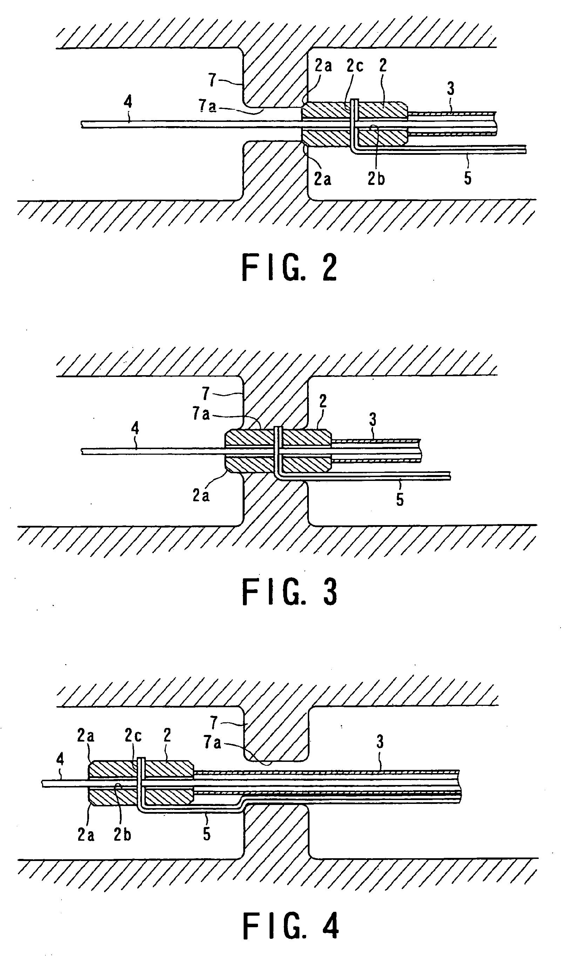

A method of using the organ anastomosing apparatus of the characters mentioned above will be described hereunder with reference to Figs. 2 to 9. It should be noted that said method does not form part of the invention, but represents and example useful for understanding the invention.

First, as shown in Fig. 2, the external end of the guide wire 4, which is to be inserted into the predetermined organ of a subject such as a patient, is inserted through the lateral hole 2b of the first magnet 2, in which the vinculum 5 is preliminarily inserted in the vertical hole 2c of the first magnet 2 at an outside of the subject's body. Then, the guide wire 4 and the first magnet 2 are inserted into the subject's body while observing an X-ray fluoroscopic screen. The following operation is also carried out while appropriately observing the X-ray fluoroscopic screen.

Then, the opening end of the tube 3 inserted into the outer (external) end of the guide wire 4 is contacted with the circumferential side surface of the first magnet 2, and then, the first magnet 2 is moved to one side of the constricted portion 7, which is one portion of a narrow region, along the guide wire 4.

Thereafter, as shown in Fig. 3, the first magnet 2 is pushed forward by the tube 3, from the circular arc circumference side of the first magnet 2, into the fistula 7a of a through hole of the constricted portion 7, and then, as shown in Fig. 4, the first magnet 2 is pushed out to the forward space of the constricted portion 7.

Next, as shown in Fig. 5, the tube 3 is withdrawn from the guide wire 4, and as shown in Fig. 6, the guide wire 4 is withdrawn from the lateral hole 2b of the first magnet 2, to temporally place the first magnet 2 at the forward space of the constricted portion 7.

Thereafter, as shown in Fig. 7, both ends of the folded vinculum 5 are pulled outward from the outside of the subject's body. Accordingly, the first magnet 2 turns with both its end surfaces (lateral circumferential sides) upwardly directed, as shown in Fig. 7, and then, one end surface in the axial direction of the first magnet 2 contacts and latches to (engages with) one end surface of the constricted portion 7. Thus, the vinculum 5 is strained, and in this state, the external end of the vinculum 5 is inserted into the longitudinal hole 6a of the second magnet 6 while keeping the tension thereof outside the subject's body, for example, and also inserted into the tube 3.

Next, as shown in Fig. 7, the opening end of the tube 3 is contacted with and pushed against the center position of one end surface in the axial direction of the second magnet 6 to thereby push the second magnet into the organ of the subject's body.

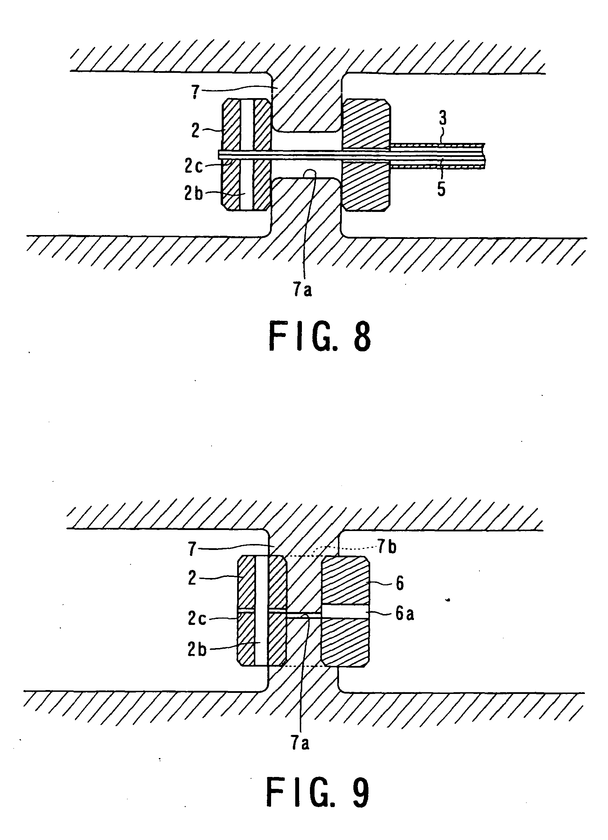

For this reason, as shown in Fig. 8, the second magnet 6 reaches and contacts with the other end surface of the constricted portion 7 through the movement along the vinculum 5.

Accordingly, as shown in Fig. 9, the second magnet 6 is attracted to the first magnet 2 by a strong magnetic force. Thus, the constricted portion 7 is strongly pinched and compressed by the pair of the first and second magnets 2 and 6. Thereafter, the tube 3 is withdrawn from the subject's body, and either one of the external ends of the vinculum 5 protruding outward from of the subject's body is pulled, that is, along the approach route or the return route, and then, the vinculum 5 is withdrawn from the longitudinal hole 6a of the second magnet 6 and the longitudinal (vertical in Fig. 1, for example,) hole 2c of the first magnet 2 so as to recover the vinculum 5 outside of the subject's body.

The first and second magnets 2 and 6, respectively, pinch and press from both sides of the constricted portion 7 for a certain period of time, eventually inducing apoptosis in the cellular structure at the pinched and pressed region of the constricted portion 7, thus forming the second through hole 7b having almost the same diameter as those of the first and second magnets 2 and 6 at the outer circumferential portion of the through hole 7a.

For this reason, the narrow fistula 7a at the constricted portion 7 is expanded to the second through hole 7b, which has a greater diameter, thus reducing or removing the constriction of the constricted portion 7. Furthermore, during the formation of the second through hole 7b, the periphery of the through hole 7b coalesces, and the new anastomosis is formed.

In addition, the cellular structure, in which apoptosis is caused by being pinched and pressed by the first and second magnets 2 and 6, is finally discharged outside of the subject's body together with the first and second magnets 2 and 6 while remaining pinched and pressed therebetween.

Therefore, according to the organ anastomosis apparatus 1 of the present invention, the first magnet 2 is pushed so as to be inserted into the fistula 7a of the narrow constricted portion 7 from the circular arc-shaped circumference side thereof, and accordingly, the first magnet can be easily pushed and inserted into the fistula 7a with a small pushing force.

Furthermore, since the second magnet 6 has a taper 2a at the peripheral rims (edges), it can be easily and smoothly inserted into the fistula 7a with a small pushing force.

In addition, since the first magnet 2 is latched by the turn-round point of the vinculum 5 at the crossbar 2d, after drawing out the guide wire 4 from the lateral hole 2b of the first magnet 2 by simply pulling one end of the vinculum 5, extending outside of the subject's body, that is, along the approach or return route, as shown in Fig. 7, the first magnet 2 can be easily and reliably controlled to rise up inside an organ and to be thereby latched to one side of the constricted portion 7.

That is, the first magnet 2 can be easily inserted into and through the fistula 7a of the constricted portion 7 without using any accessories, tool or like, and after passing through the first magnet 2, it can be easily and reliably controlled to rise up and to be latched to one side of the constricted portion 7.

Furthermore, since the internal end of the vinculum 5 is not secured to one end of the first magnet 2, but the turn-round point of the vinculum 5 is simply latched to or engaged with the crossbar 2d of the first magnet 2, the vinculum 5 can be easily recovered outside the subject's body, without remaining in the body (organ), merely by pulling the other one ends (external end) of the vinculum 5, on the approach route or back-haul route, extending outside the subject's body.

Still furthermore, the outer surfaces of the first and second magnets 2 and 6 are coated with an acid-resistant membrane or a thrombus-preventing membrane. Thus, deterioration or degradation of these magnets caused by oxidation due to humor (body fluid) in the organ of the subject's body can be prevented or reduced. In addition, the generation of a thrombus due to the first and second magnets 2 and 6 in blood can be prevented.

Still furthermore, the first and second magnets 2 and 6 are made of a rare earth element, so that the magnetic force of the first and second magnets 2 and 6 can be strengthened, and therefore, even if the constricted portion 7 or anastomosis portion has a large thickness, the attraction between the first magnet 2 and the second magnet 6 can be easily and reliably achieved, and these magnets can be effectively reduced in size and thickness thereof.

It is to be noted that although the foregoing embodiment exemplifies a case applying the organ anastomosing apparatus 1 to the treatment of the constricted portion 7, the anastomosing apparatus 1 according to the present invention can be used to form an anastomosis portion.

In addition, one end of the vinculum 5 may be secured to the center position of one side in the axial direction of the first magnet 2. In this case also, by simply pulling the vinculum 5 toward the outside, the first magnet 2 can be easily and reliably controlled so as to be latched to the erected constricted portion 7 in the organ, and the second magnet 6 can be moved to a predetermined position of the organ. In the present case, the vinculum 5 is formed of a material capable of being dissolved by the body humor in the organ so as to prevent the vinculum 5 from remaining in the organ.

In addition, by placing a marker made of an X-ray non-transmitting material indicating the magnetic pole of at least one of the first and second magnets 2 and 6, the magnetic pole of the first and second magnets 2 and 6 inserted in an organ can be confirmed by monitoring an X-ray fluoroscopic screen. Accordingly, attraction between the first and second magnets 2 and 6 can be easily and reliably performed.

Furthermore, although the foregoing embodiment exemplifies a case using the tube 3 as a moving means, the moving means may be an endoscope or an external induction magnet or the like, not shown, which allows the first and second magnets 2 and 6 to move to a predetermined position in an organ. The induction magnet described above may be a member to attract the first and second magnets 2 and 6 with a magnetic force from outside the subject's body, as far as it attracts the magnets and moves the induction magnet outside of the subject' body, and hence, a superconducting magnet may be preferably used. Further, although the foregoing embodiment exemplifies a case where the taper 2a is formed on the end surface of the first magnet 2, such taper 2a may be eliminated.

### Industrial Applicability

As described hereinbefore, the present invention enables an anastomosis portion or a constricted portion to be reduced or removed by physically expanding the narrow through hole thereof by removing peripheral rims around the narrow through hole of the anastomosis portion or the constricted portion of a subject's body.

## Claims

1. An organ anastomosing apparatus (1) comprising:
a flexible guide wire (4) to be inserted into an organ;
a first magnet (2) formed in a disc shape;
a vinculum (5) secured at a center position of one end surface of the first magnet (2) in an axial direction thereof; and
a second magnet (6) provided with a through hole (6a) in which the vinculum (5) is inserted; **characterized in that** the apparatus further comprises a moving member (3) for moving the first and second magnets (2; 6), and that the first magnet (2) is provided with a radial through hole (26) so as to slidably insert the guide wire (4).

2. An organ anastomosing apparatus (1) according to claim 1,
wherein said first magnet (2) is provided with a latch member for engaging a turn-around portion of the vinculum (5) when the vinculum (5) is folded in two portions.

3. An organ anastomosing apparatus (1) according to claim 1 or 2,
wherein said vinculum (5) is secured to a center portion of one end surface of the first magnet (2) in an axial direction thereof.

4. An organ anastomosing apparatus (1) according to claim 3,
wherein said vinculum (5) is made of a material which is dissolved by humor in the organ of a subject.

5. An organ anastomosing apparatus according to any one of Claims 1 to 4,
wherein said first magnet (2) is chamfered at corner portions of end surfaces in the axial direction thereof.

6. An organ anastomosing apparatus according to any one of Claims 1 to 5,
wherein said moving member (3) is composed of a tubular member (3) movably mounted to the guide wire (4), said tubular member (3) pushing front end portions of lateral circumferential sides of the first and second magnets (2 ; 6).

7. An organ anastomosing apparatus (1) according to any one of Claims 1 to 6,
wherein either one of the first and second magnets (2 ; 6) is provided with a marker made of an X-ray non-transmitting material indicating a magnetic pole of the magnet.

## Patentansprüche

1. Organanastomosevorrichtung (1), umfassend:
einen in ein Organ einzusetzenden flexiblen Führungsdraht (4),
einen ersten Magneten (2), der in einer Scheibenform ausgebildet ist,
ein Band (5), das an einer Mittelposition einer Endfläche des ersten Magneten (2) in einer axialen Richtung davon angebracht ist, und
einen zweiten Magneten (6), der mit einem Durchgangsloch (6a) ausgestattet ist, in welches das Band (5) eingesetzt ist, **dadurch gekennzeichnet, dass**
die Vorrichtung ferner ein Bewegungselement (3) zum Bewegen des ersten und des zweiten Magneten (2; 6) umfasst und dass der erste Magnet (2) so mit einem radialen Durchgangsloch (2b) ausgestattet ist, dass der Führungsdraht (4) gleitend eingesetzt ist.

2. Organanastomosevorrichtung (1) nach Anspruch 1, bei welcher der erste Magnet (2) mit einem Fixierelement zum Ineingriffnehmen eines Schlaufenabschnitts des Bands (5), wenn das Band (5) in zwei Abschnitte gefaltet ist, ausgestattet ist.

3. Organanastomosevorrichtung (1) nach Anspruch 1 oder 2, bei welcher das Band (5) an einem Mittelabschnitt einer Endfläche des ersten Magneten (2) in einer axialen Richtung davon angebracht ist.

4. Organanastomosevorrichtung (1) nach Anspruch 3, bei welcher das Band (5) aus einem Material hergestellt ist, dass durch die Körperflüssigkeit in dem Organ eines Lebewesens aufgelöst wird.

5. Organanastomosevorrichtung nach einem der Ansprüche 1 bis 4, bei welcher der erste Magnet (2) an Eckenabschnitten von Endflächen in dessen axialer Richtung abgeschrägt ist.

6. Organanastomosevorrichtung nach einem der Ansprüche 1 bis 5, bei der das Bewegungselement (3) aus einem röhrenförmigen Element (3) zusammengesetzt ist, das bewegbar an dem Führungsdraht (4) angebracht ist, wobei das röhrenförmige Element (3) vordere Endabschnitte von seitlichen Umfangsseiten des ersten und des zweiten Magneten (2; 6) schiebt.

7. Organanastomosevorrichtung (1) nach einem der Ansprüche 1 bis 6, bei der einer des ersten und des zweiten Magneten (2; 6) mit einem Marker ausgestattet ist, der aus einem für Röntgenstrahlen undurchlässigen Material ausgebildet ist und einen magnetischen Pol des Magneten anzeigt.

## Revendications

1. Appareil d'anastomose d'organes (1) composé :
d'un passe-fil flexible (4) à insérer dans l'organe,
d'un premier aimant (2) en forme de disque,
d'un vinculum (5) sécurisé à la position centrale d'une surface d'extrémité du premier aimant (2) dans la direction axiale appropriée et
d'un deuxième aimant (6) fourni avec un trou débouchant (6a), dans lequel est inséré le vinculum (5) et dont la **caractéristique est que** l'appareil est composé encore d'un élément mobile (3) permettant de déplacer le premier et le deuxième aimant (2; 6) et que le premier aimant (2) est fourni avec un trou débouchant radial (2b) de manière à insérer le passe-fil (4) en le glissant.

2. Appareil d'anastomose d'organes (1) conforme à la revendication 1, où l'on dit que le premier aimant (2) est fourni avec un élément de serrage pour engager la partie tournante du vinculum (5) quand le vinculum (5) est plié en deux morceaux.

3. Appareil d'anastomose d'organes (1) conforme à la revendication 1 ou 2, où l'on dit que le vinculum (5) est sécurisé sur la partie centrale d'une extrémité de la surface du premier aimant (2) dans la direction axiale appropriée.

4. Appareil d'anastomose d'organes (1) conforme à la revendication 3, où l'on dit que le vinculum (5) est fabriqué à partir d'un matériau dissous par l'humeur dans l'organe du sujet.

5. Appareil d'anastomose d'organes conforme à n'importe quelle revendication de 1 à 4, où l'on dit que le premier aimant (2) est chanfreiné aux deux zones en coin de l'extrémité des surfaces dans la direction axiale appropriée.

6. Appareil d'anastomose d'organes conforme à n'importe quelle revendication de 1 à 5, où l'on dit que l'élément mobile (3) est composé d'un élément tubulaire (3) monté de manière amovible sur le passe-fil (4), ledit élément tubulaire (3) poussant les parties frontales des côtés circonférentiels latéraux du premier et du deuxième aimant (2; 6).

7. Appareil d'anastomose d'organes (1) conforme à n'importe quelle revendication de 1 à 6, où l'on dit soit le premier aimant ou le deuxième aimant (2; 6) sont fournis avec un marqueur de rayon X ne transmettant pas de matériel indiquant un pôle magnétique de l'aimant.
